# EUROPEAN PATENT APPLICATION

(11) **EP 1 815 787 A1**
(43) Date of publication of application: **08.08.2007**
(21) Application number: 05755827.2
(22) Date of filing: 30.06.2005
(51) Int. Cl.: A61B 5/0245, A61B 5/00, A61B 5/11

(54) **HEARTBEAT/RESPIRATION SENSOR AND BODY MONITOR EMPLOYING SAME**

(30) Priority: 02.11.2004 JP 2004319422
(71) Applicant: A.T. Labo, Co., Ltd., Akita-shi, Akita 010-0061 (JP)
(72) Inventor: SATO, Shinichi, 0100834 (JP); YAMADA, Katsuya, 0101101 (JP); INAGAKI, Nobuya, 0100873 (JP); ISHIDA, Akira, Akita-shi, Akita 0100041 (JP)
(74) Representative: Burbaud, Eric
(86) International application number: PCT/JP2005/012068
(87) International publication number: WO 2006/048960

(57) **Abstract**

Heartbeat/respiration sensor 4 comprises flexible vibration transmitting plate 1 formed with a plurality of air vents 3, and piezoelectric transducer 2 mounted on vibration transmitting plate 1.

## Description

### TECHNICAL FIELD

The present invention relates to a heartbeat/respiration sensor for detecting the heartbeat, respiration and the like of a subject such as a newborn infant, and a body monitor employing the same.

### BACKGROUND ART

A newborn infant in bad health is placed in an incubator and treated in an intensive care unit of a hospital. In this event, electrodes of an electrocardiograph are attached on the newborn infant to measure its heart rate and the like. However, since the newborn infant's sebum must be removed with alcohol or the like before attaching the electrodes of the electrocardiograph, a problem of complexity is involved in electrocardiogram-based measurements of heart rate and respiration rate. Also, since the newborn infant has relatively weak skin, it is sometimes the case that the newborn infant will develop rashes on the skin after the electrodes of the electrocardiograph have been attached for a long time, and the skin can peel off at the time when the electrodes of the electrocardiograph are removed. Further, the electrodes of the electrocardiograph can come off the newborn infant if the newborn infant moves during electrocardiogram-based measurements. Accordingly, the heart rate and respiration rate of the newborn infant, in particular, are preferably measured by a device which can measure them in a noninvasive and unbounded state.

Conventionally, devices for measuring vital data such as the heart rate, respiration rate and the like of a subject in a noninvasive and unbounded state are known from WO03/067967A1, JP10-14889A, and JP2002-52009A.

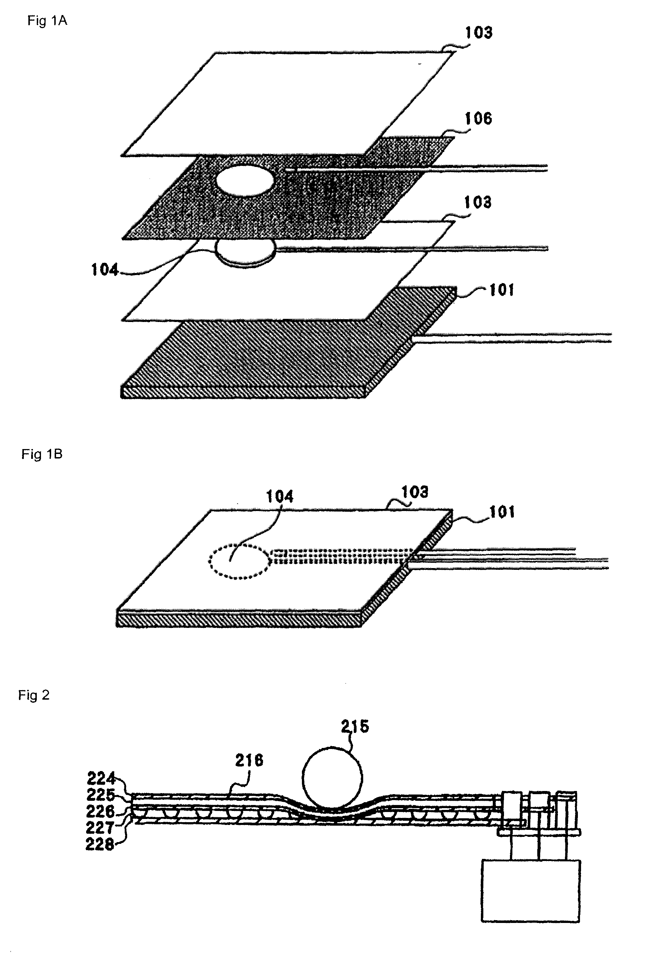

WO03/067967A1 discloses a body heat sustaining device having a heartbeat/respiration rate detecting function for small animals such as a mouse, and a small animal heartbeat/respiration rate measuring system using the same. Figs. 1A and 1 B are perspective views illustrating the body heat sustaining device having a heartbeat/respiration rate measuring function for small animals, disclosed in WO03/067967A1. As illustrated in Figs. 1A and 1B, the body heat sustaining device comprises flat heater 101, and pressure sensitive sensor 104 and spacer 106 sandwiched between insulating sheets 103 and placed on flat heater 101. In addition to sustaining the body heat of a small animal by flat heater 101, pressure sensitive sensor 104 can detect the heartbeat/respiration rate of the small animal.

JP10-14889A in turn discloses a vital signal detector for detecting vital signals related to the weight, heartbeat, respiration, active mass, life condition and the like. Fig. 2 is a cross-sectional view of a main portion of a sensor sheet in the vital signal detector disclosed in JP10-14889A. Sensor sheet 216 illustrated in Fig. 2 comprises first conductive layer 224, dielectric elastic insulating layer 225, second conductive layer 226, insulating spacer 227, and third conductive layer 228, laminated in this order from above. According to this sensor sheet 216, movements of living body 215 on sensor sheet 216 cause changes in thickness of elastic insulating layer 225 to make electrostatic capacitances, that are formed between first and second conductive layers 224, 226 and elastic insulating layer 225 placed therebetween, fluctuate. Movements of the living body (heartbeat, respiration rate and the like) are detected by detecting fluctuations in the electrostatic capacitances. Detecting whether pressure from the weight of a living body is applied to sensor sheet 216 is determined by whether or not insulating spacer 227 is collapsed to bring conductive layers 226, 228 into contact with each other, i.e., whether or not conduction occurs between conductive layers 226, 228. When a pressure sensitive resistive layer is used instead of insulating spacer 227, detailed weight pressure data can be provided in accordance with the weight.

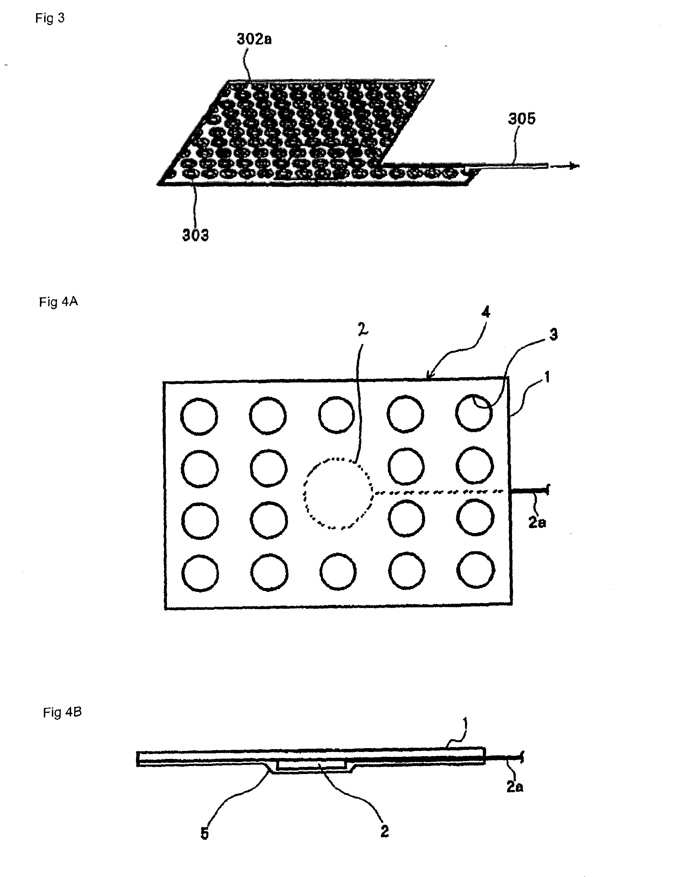

JP2002-52009A in turn discloses a subject data measuring air mat for measuring vital data of a subject in bed. Fig. 3 is a perspective view illustrating the air mat disclosed in JP2002-52009A. As illustrated in Fig. 3, the air mat comprises joints 303 at which upper textile 302a is adhered to a lower textile (not shown), formed at a plurality of locations at predetermined intervals. Air introduction tube 305 which extends from an inner space of the air mat is in communication with a pressure sensor (not shown), such that this pressure sensor detects changes in the internal pressure of the air mat to measure vital data of the subject.

Particular characteristics are associated with a newborn infant, as compared with the adult, such as a smaller body (height, weight), a higher susceptibility to rashes due to perspiration, and the like. Accordingly, heartbeat/respiration sensors that are, in particular, used for newborn infants must have the ability to highly sensitively make detections over a long period of time, even in an incubator, and must be able to prevent the newborn infants from developing rashes and the like.

However, the body heat sustaining device that has a heartbeat/respiration rate detecting function for small animals disclosed in International Publication No. 03/067967 pamphlet will give discomfort to a newborn infant placed on a flat heater, due to its rigid structure, and prevent the newborn infant from having rest and a sound sleep. Consequently, the body heat sustaining device fails to stably measure the correct heart rate and respiration rate of the newborn infant over a long time. Also, from the fact that the overall body heat sustaining device is thick due to the large thickness of the flat heater, the body heat sustaining device will get snagged on a towel which is turned over when attempts to insert the body heat sustaining device between the bed and the towel on which the newborn infant is lying in an incubator. Therefore, in such a situation, the body heat sustaining device must be installed in the incubator while the newborn infant is lifted and held in the arms of a hospital staff. As such, it cannot be said that the body heat sustaining device generally excels in user-friendliness for doctors, nurses and the like when it is used for a newborn infant in an incubator.

The sensor sheet disclosed in JP10-14889A in turn is configured to detect movements (heart rate, respiration rate and the like) of a living body as it is compressed. It is therefore possible to relatively highly sensitively detect such movements when a newborn infant is directly in contact with the sensor sheet which is installed on a rigid base. However, in doing so, the newborn infant is given discomfort and therefore prevented from having rest and a sound sleep, therefore the sensor sheet is actually installed on a soft comforter, with a towel interposed between the sensor sheet and subject (newborn infant). In this event, however, since movements of the living body are absorbed by the comforter, towel and the like, the detection sensitivity of the sensor sheet is largely degraded. From such circumstances, it can be said that the sensor sheet of Patent Document 2 is not suitable for use in applications which involve capturing respiration/heartbeat data from a newborn infant in an incubator.

In this connection, vent holes may be formed in the body heat sustaining device (WO03/067967A1) and sensor sheet (JP10-14889A) for providing ventilation, in which case available vent holes are limited in size and number because large vent holes or a large number of vent holes would cause the flat heater to generate an insufficient amount of heat which would compromise the heat insulation effect of the body heat sustaining device, or would correspondingly reduce the sensing area of the sensor sheet and cause it to have lower detection accuracy. Thus, the body heat sustaining device and sensor sheet described above may fail to provide sufficient ventilation when they are used for the newborn infant because of reasons related to the structure. In addition, when they are formed with vent holes, the hole formed surfaces must be covered with a water-proof film for purposes of protecting the hole formed surfaces from humidity and the like, in order to prevent malfunctions due to humidity and the like or to prevent electrodes and the like from being exposed, resulting in a corresponding increase in manufacturing cost.

Also, the air mat of JP2002-52009A has a thickness of at least several centimeters because the air mat must have sufficient air volume to ensure that it does not collapse if a subject lies thereon. Thus, such an air mat is not suitable for installation and use in a small incubator. In addition, it is virtually impossible to insert such an air mat between a bed and a sheet by turning over a towel which is laid underneath the newborn infant who is lying in the incubator.

### DISCLOSURE OF THE INVENTION

It is therefore an object of the present invention to provide a heartbeat/respiration sensor in a simple configuration which is capable of highly sensitively detecting a heart rate and a respiration rate in a noninvasive and unbounded state without causing discomfort to a subject (in particular, a newborn infant), even if used for a long time, and a body monitor which employs the sensor.

To achieve the above object, a heartbeat/respiration sensor of the present invention comprises a flexible vibration transmitting plate formed with a plurality of vent holes therethrough, and a piezoelectric transducer mounted on the vibration transmitting plate.

The piezoelectric transducer per se is highly sensitive and is capable of detecting vital signals, and the vibration transmitting plate serves to extend the range in which vital signals can be detected by the piezoelectric transducer. Therefore, according to the heartbeat/respiration sensor of the present invention, the vibration transmitting plate receives a vital signal emitted from a subject in proximity to the heartbeat/respiration sensor as sound, vibrations or the like, and the piezoelectric transducer detects vibrations generated in the vibration transmitting plate by the vital signal, thereby making it possible to widely and highly sensitively detect the vital signal of the subject in a noninvasive and unbounded state. Also, since the heartbeat/respiration sensor of the present invention is configured such that the vibration transmitting plate receives the vital signal emitted from the subject, the heartbeat/respiration sensor need not be necessarily installed and used on a rigid base, unlike the conventional sensor seat and the like. Therefore, the heartbeat/respiration sensor can highly sensitively detect the vital signal even if it is installed on a soft comforter, or even it is wrapped with a towel or the like, or even the subject remains with his/her clothes on.

Also, the heartbeat/respiration sensor of the present invention has the vibration transmitting plate formed with a plurality of vent holes. Unlike the conventional sensor sheet and the like, the vibration transmitting plate in the heartbeat/respiration sensor of the present invention is not provided with a heater, electrode wires or the like, therefore the vent holes which can be formed through the vibration transmitting plate are not limited thereby in number or size. Thus, in the configuration of the present invention, the vibration transmitting plate can be formed with vent holes to such a degree that provide sufficient ventilation can be provided. Accordingly, even if a subject perspires when he/she is lying on the heartbeat/respiration sensor, humidity is radiated through the vent holes, thus protecting the subject from rashes and the like due to frowstiness.

Further, because of its flexibility, the vibration transmitting plate deforms in response to movements of a subject such as rolling-over when he is lying on the heartbeat/respiration sensor, so that the heartbeat/respiration sensor of the present invention does not cause discomfort to the subject. It is therefore possible to detect a vital signal for a long time without impeding the rest and sleep of the subject.

In addition, the heartbeat/respiration sensor of the present invention is made up of the vibration transmitting plate and piezoelectric transducer mounted thereto, and can be formed in a simple and thin sheet shape. Thus, the heartbeat/respiration sensor can be inserted, for example, by turning over a towel which is underneath a newborn infant who is lying in an incubator.

Further, the vibration transmitting plate may have rounded corners. Consequently, when the heartbeat/respiration sensor is inserted by turning over a towel which is underneath a newborn infant who is sleeping in an incubator, the heartbeat/respiration sensor can be designed so that it is less likely to get snagged on the towel.

Also, an alignment line may be drawn on the vibration transmitting plate for indicating the position at which the piezoelectric transducer is mounted. In this way, the piezoelectric transducer can be visually positioned more correctly at a desired measurement site such as below the chest of a subject or the like.

Further, the heartbeat/respiration sensor may be encased in a cover made of terry cloth, wherein an alignment line may be drawn on the cover for indicating the position of the piezoelectric transducer when the cover encases the heartbeat/respiration sensor. The heartbeat/respiration sensor deforms in conformity with the shape of a newborn infant due to the weight of the newborn infant, and the cover serves as a buffer between the heartbeat/respiration sensor and newborn infant with its cushioning effect, so that the heartbeat/respiration sensor will not cause discomfort to the newborn infant even if it is used while encased in the cover. Also, since the piezoelectric transducer is sensitive enough to detect not only pressure and vibrations but also sound, the heartbeat/respiration sensor can sufficiently detect the heartbeat and respiration even when it is encased in the cover and placed below the body of the newborn infant. Further, when the heartbeat/respiration sensor is used in this way, ventilation spaces are formed around heartbeat/respiration sensor 4 by the cover. Thus, humidity caused by perspiration of the newborn infant can be diffused through these spaces and vent holes of the heartbeat/respiration sensor, so that the newborn infant can be prevented from developing rashes on the skin. Further, since the alignment line is drawn on the cover, the piezoelectric transducer can be more correctly positioned at a desired measurement site such as below the chest of the subject or the like even if the cover encases the heartbeat/respiration sensor.

Also, a body monitor of the present invention comprises the heartbeat/respiration sensor of the present invention, a filter circuit for separating a cardiac sound signal and a respiration signal from an output signal of the heartbeat/respiration sensor, and a monitor for displaying the cardiac sound signal and the respiration signal.

According to the body monitor of the present invention, the cardiac sound signal and respiration signal are extracted from the vital signal detected by the heartbeat/respiration sensor and are displayed on the monitor, thus making it possible to monitor a subject for the heart rate and respiration rate by monitoring these signals on the monitor. Further, sleep data is generated by continuously recording the cardiac sound signal for the period during which the newborn infant is asleep, and includes a large amplitude signal due to movements of the subject, cardiac sound signal, mute signal and the like. Accordingly, by analyzing these signals, a simple analysis can be made on the sleeping state and sleeping pattern of the subject.

### BRIEF DESCRIPTION OF THE DRAWINGS

[Fig. 1A]
   Fig. 1A is an exploded perspective view illustrating a conventional body heat sustaining device having a function for detecting the heartbeat/respiration rate of small animals.
[Fig. 1B]
   Fig. 1B] is a perspective view illustrating the conventional body heat sustaining device having a function for detecting the heartbeat/respiration rate of small animals.
[Fig. 2]
   Fig. 2 is a cross-sectional view of a main portion of a sensor sheet in a conventional vital signal detector.
[Fig. 3]
   Fig. 3 is a perspective view illustrating a conventional subject data measuring air mat for measuring the vital data of a subject in bed.
[Fig. 4A]
   Fig. 4A is a plan view illustrating one embodiment of a heartbeat/respiration sensor according to the present invention.
[Fig. 4B]
   Fig. 4B is a cross-sectional view illustrating one embodiment of the heartbeat/respiration sensor according to the present invention.
[Fig. 5]

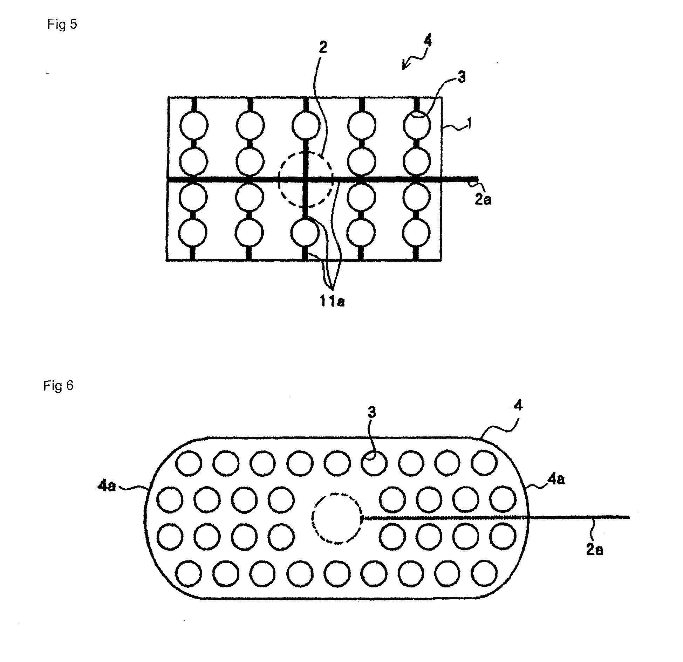

   Fig. 5 is a plan view illustrating one exemplary modification to the heartbeat/respiration sensor illustrated in Fig. 4.
[Fig. 6]

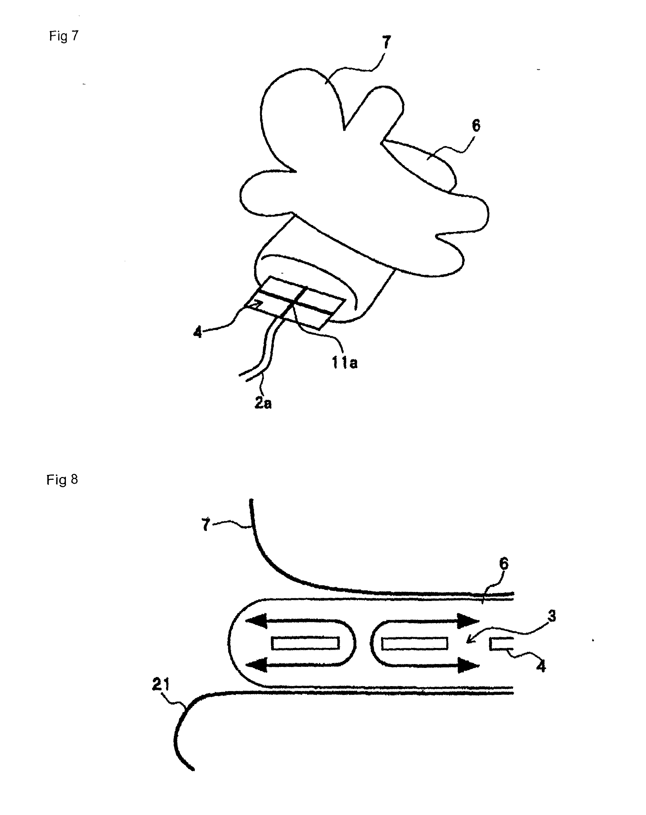

   Fig. 6 is a plan view illustrating another exemplary modification to the heartbeat/respiration sensor illustrated in Fig. 4.
[Fig. 7]
   Fig. 7 is a diagram showing how the heartbeat/respiration sensor is wrapped with a towel and placed below a newborn infant.
[Fig. 8]
   Fig. 8 is a diagram showing how the heartbeat/respiration sensor is wrapped with a towel and placed below a newborn infant.
[Fig. 9]

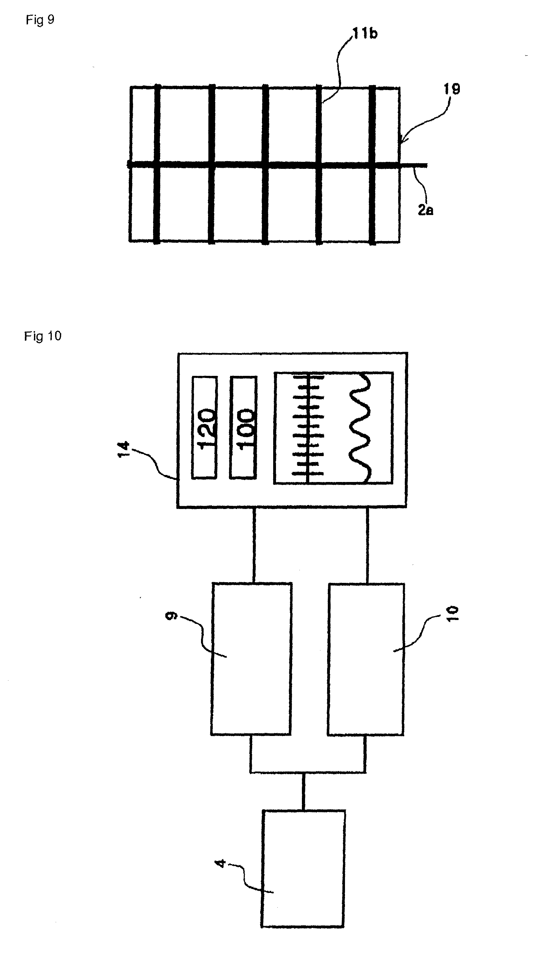

   Fig. 9 is a diagram showing a towel cover used to the heartbeat/respiration sensor.
[Fig. 10]
   Fig. 10 is a block diagram illustrating a body monitor which enables a heart rate, respiration rate and the like to be monitored based on a signal generated by the heartbeat/respiration sensor.
[Fig. 11]

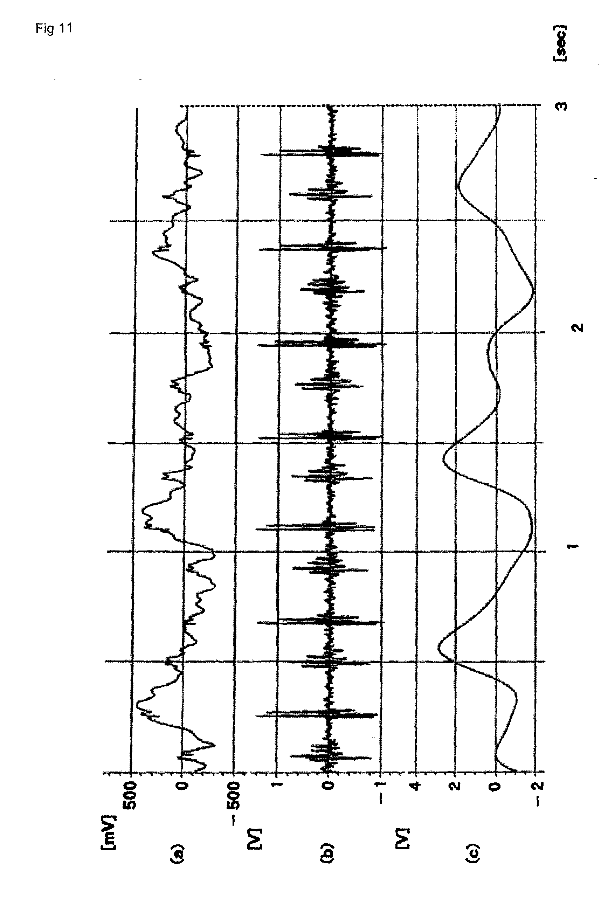

   Fig. 11 is a diagram showing the waveforms of the signal generated from the heartbeat/respiration sensor, a cardiac sound signal and a respiration signal.
[Fig. 12]

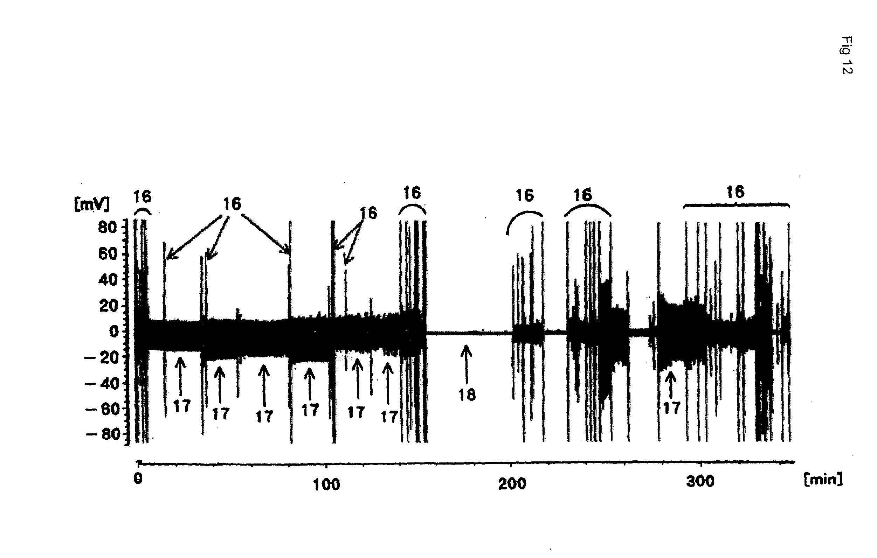

   Fig. 12 is a diagram showing sleep data generated by continuously recording a cardiac sound signal during the period during which the newborn infant is asleep.

### BEST MODE FOR CARRYING OUT THE INVENTION

Next, an embodiment of the present invention will be described with reference to the drawings.

### <Heartbeat/Respiration Sensor>

Figs. 4A and 4B are diagrams illustrating one embodiment of a heartbeat/respiration sensor according to the present invention, where Fig. 4A is a plan view thereof, and Fig. 4B is a cross-sectional view thereof.

As illustrated in Figs. 4A and 4B, heartbeat/respiration sensor 4, which is a heartbeat/respiration sensor of this embodiment, comprises flexible vibration transmitting plate 1, and piezoelectric transducer 2 mounted on a central region on the back side of vibration transmitting plate 1 with an adhesive or the like. A plurality of vent holes 3 are formed through an area of vibration transmitting plate 1 except for the area in which piezoelectric transducer 2 is mounted. Further, insulating film 5 is formed on the back side of vibration transmitting plate 1 for covering at least piezoelectric transducer 2 and lead 2a extending therefrom. This insulating film 5 is bored in portions corresponding to vent holes 3 so as not to cover vent holes 3 of vibration transmitting plate 1.

Vibration transmitting plate 1 is made of plastic resin or the like shaped in the form of a thin sheet having the following dimensions: Height 200 mm; Width 100 mm; Thickness 1 mm, by way of example. In this connection, the thickness can be decided as appropriate in accordance with the hardness of the material of vibration transmitting plate 1.

Piezoelectric transducer 2 in turn is circular in shape, and has a diameter of 27 mm and a thickness of 0.5 mm, by way of example. Therefore, heartbeat/respiration sensor 4 of this embodiment has a thickness of approximately 1.5 mm in the area in which piezoelectric transducer 2 is mounted, and approximately 1 mm in the area in which piezoelectric transducer 2 is not mounted, even taking into consideration the thickness of insulating film 5. In this connection, piezoelectric transducer 2 used herein can be made, for example, of piezo-ceramic.

Heartbeat/respiration sensor 4 of this embodiment configured as described above, when used, is installed, for example, between a towel which is placed below a newborn infant lying in an incubator and an underlying bed comforter. Heartbeat/respiration sensor 4 is preferably installed such that it is positioned below the chest of the newborn infant so that it can satisfactorily detect the heartbeat and respiration of the newborn infant. With heartbeat/respiration sensor 4 installed in such a state, vibration transmitting plate 1 is vibrated by the cardiac sound and respiration sound of the newborn infant, and its vibrations are transmitted to piezoelectric transducer 2. Piezoelectric transducer 2 converts distortions produced therein due to the vibrations into an electric signal which is supplied to a body monitor, later described, through lead 2a. In this way, since heartbeat/respiration sensor 4 of this embodiment is configured to detect the cardiac sound and respiration sound of the newborn infant by converting vibrations generated in vibration transmitting plate 1 into an electric signal by piezoelectric transducer 2, the heartbeat and respiration can be sensitively detected even if the sensor is installed on a soft comforter, unlike the conventional sensor sheet and air mat which detect cardiac sound and respiration sound based on the pressure applied by a subject.

It should be noted that if piezoelectric transducer 2 alone is installed below a newborn infant, cardiac sound and respiration sound can be detected only in the area covered by the size of piezoelectric transducer 2. If the newborn infant moves even a little, such as rolling over in the incubator, piezoelectric transducer 2 will fail to detect the cardiac sound and respiration sound. Thus, when one attempts to use piezoelectric transducer 2 alone, larger piezoelectric transducer 2 must be used in order to satisfactorily make detections in a wider area. However, since piezoelectric transducer 2 is not flexible, large piezoelectric transducer 2 installed under a newborn infant will cause discomfort to the newborn infant. In contrast, the employment of flexible vibration transmitting plate 1 in this embodiment enables relatively small piezoelectric transducer 2 to detect cardiac sound and respiration sound over a wider range. Further, in this event, the newborn infant will not be caused any discomfort because piezoelectric transducer 2 is relatively small.

Also, since heartbeat/respiration sensor 4 of this embodiment has a thickness of approximately 1.5 mm even in the thickest portion as described above, a towel placed under a newborn infant lying in an incubator can be readily turned over to insert heartbeat/respiration sensor 4 between the bed and towel. Further, since vibration transmitting plate 1 is flexible, vibration transmitting plate 1 deforms in response to any movements of the newborn infant lying on heartbeat/respiration sensor 4, such as roll-over. Thus, the newborn infant will not be caused any discomfort. In addition, a plurality of vent holes 3 formed through vibration transmitting plate 1 serve to pass humidity between the front and back surfaces of heartbeat/respiration sensor 4, so that even if heartbeat/respiration sensor 4 is disposed under a newborn infant, the newborn infant will not have rashes due to perspiration during night and the like.

Unlike the conventional sensor sheet and the like, vibration transmitting plate 1 of this embodiment is simple in structure without any heating element, wiring electrodes and the like disposed thereon, so that vent holes 3 can be formed freely except for the area in which piezoelectric transducer 2 is mounted. In this connection, means which can be formed in vibration transmitting plate 1 for purposes of ventilation is not limited to the form of vent holes 3, but vibration transmitting plate 1 may be alternatively configured, for example, in a lattice or a net form to allow for ventilation.

Also, from the fact that heartbeat/respiration sensor 4 of this embodiment has a simple configuration made up of vibration transmitting plate 1 and from the fact that piezoelectric transducer 2, and piezoelectric transducer 2 is further protected by insulating film 5, heartbeat/respiration sensor 4 can be readily disinfected with alcohol. As such, heartbeat/respiration sensor 4 of this embodiment can be readily kept clean, and is suitable for use in an incubator in which there is a particular requirement for cleanness. Further, when vibration transmitting plate 1 and insulating film 5 are made of heatproof and chemical proof materials, other sterilizing/disinfecting means can be applied as well.

Fig. 5 is a plan view illustrating one exemplary modification to the heartbeat/respiration sensor illustrated in Figs. 4A and 4B. On the top side of heartbeat/respiration sensor 4 illustrated in Fig. 5, alignment line 11a is drawn for use as a mark indicative of the position of piezoelectric transducer 2 in heartbeat/respiration sensor 4. In this way, piezoelectric transducer 2 can be visually positioned more correctly at a desired measurement site, such as below the chest of a newborn infant. In this connection, alignment line 11a may be drawn in a relatively deep color, in which case even when heartbeat/respiration sensor 4 is inserted between a towel and a sheet, alignment line 11 a can be readily viewed even therethrough.

Fig. 6 is a plan view illustrating another exemplary modification to the heartbeat/respiration sensor illustrated in Figs. 4A and 4B. Heartbeat/respiration sensor 4 illustrated in Fig. 6 has rounded corners 4a. In this way, when heartbeat/respiration sensor 4 is inserted under a towel or a sheet, heartbeat/respiration sensor 4 is less likely to get snagged thereon, thus making it possible to more readily insert heartbeat/respiration sensor 4 under a towel or a sheet.

Fig. 7 is a perspective view illustrating how the heartbeat/respiration sensor of this embodiment is wrapped with a towel and disposed below a newborn infant. In this connection, Fig. 7 illustrates an example which employs heartbeat/respiration sensor 4 of the form illustrated in Fig. 5.

As described above, since heartbeat/respiration sensor 4 of this embodiment is thin and flexible, it can be wrapped with towel 6 and placed and used below the body of newborn infant 7. Heartbeat/respiration sensor 4 deforms in conformity with the shape of newborn infant 7 due to the weight of newborn infant 7, and towel 6 serves as a buffer between heartbeat/respiration sensor 4 and newborn infant 7 with its cushioning effect, so that heartbeat/respiration sensor 4 will not cause discomfort to newborn infant 7 even if it is used in this way. Also, since piezoelectric transducer 2 is sensitive enough to detect not only pressure and vibrations but also sound, heartbeat/respiration sensor 4 can sufficiently detect the heartbeat and respiration even when it is wrapped with towel 6 and placed below the body of newborn infant 7.

Further in this event, ventilation spaces are formed around heartbeat/respiration sensor 4 by towel 6, as illustrated in Fig. 8. Thus, humidity caused by perspiration of newborn infant 7 can be diffused as indicated by arrows through these spaces and vent holes 3 of heartbeat/respiration sensor 4, so that rashes can be prevented from developing on the skin of newborn infant 7.

Further, instead of wrapping heartbeat/respiration sensor 4 with towel 6, dedicated towel cover 19 may be used as illustrated in Fig. 9. This towel cover 19 has been previously formed in a bag shape for receiving heartbeat/respiration sensor 4. When heartbeat/respiration sensor 4 is wrapped with towel 6, the ventilation effect and detection of vital signals can be unstable depending on how sensor 4 is wrapped, but such a problem can be prevented by using of dedicated towel cover 19. Further, alignment line 11 b is drawn on the surface of towel cover 19. Alignment line 11 b of towel cover 19 indicates the position of piezoelectric transducer 2 when heartbeat/respiration sensor 4 is encased in towel cover 19. Therefore, even when towel cover 19 is used, piezoelectric transducer 2 of heartbeat/respiration sensor 4 can be placed at a desired position relative to newborn infant 7.

### <Body Monitor>

Fig. 10 is a block diagram illustrating a body monitor which enables the heart rate, respiration rate and the like to be monitored based on a signal generated from the heartbeat/respiration sensor described above.

The body monitor illustrated in Fig. 10 comprises two band-pass filters 9, 10, each of which receives a signal generated from heartbeat/respiration sensor 4, and monitor 14 for displaying the waveforms of the signals which have passed through respective band-pass filters 9, 10. Band-pass filter 9 has characteristics to pass therethrough a frequency band of a human's cardiac sound signal, while band-pass filter 10 has characteristics to pass therethrough a frequency band of a human's respiration signal. Therefore, when the signal (Fig. 11(a)) generated from heartbeat/respiration sensor 4 passes through band-pass filter 9, a cardiac sound signal (Fig. 11(b)) is separated therefrom, and when the signal passes through band-pass filter 10, a respiration signal (Fig. 11(c)) is separated therefrom. Monitor 14 displays the waveforms of these cardiac sound signal and respiration signal, together with the heart rate and respiration rate per unit time, derived from these waveforms. Since I-sound and II-sound can be clearly observed according to this cardiac sound signal, the body monitor can be applied to medical research through analysis of cardiac functions.

Also, the body monitor of this embodiment can be used not only for newborn infants but also for adults, elderly persons, or cared persons. For example, with heartbeat/respiration sensor 4 placed below the body of a subject at bedtime, the subject can be monitored for heart rate and respiration rate, and simple analysis can be made on the subject's sleeping state and sleeping pattern.

The heart rate and respiration rate of a subject can be acquired by appropriately monitoring the waveforms of the cardiac sound signal and respiration signal, as well as the heart rate and respiration rate displayed on monitor 14. Also, the sleeping state and sleeping pattern can be analyzed based on data which includes the cardiac sound signal (Fig. 11 (b)) continuously recorded for the period during which the subject is asleep. The cardiac sound signal is produced by passing the signal (Fig. 11(a)) generated from heartbeat/respiration sensor 4 through band-pass filter 9. Fig. 12 shows sleep data which is provided by continuously recording the cardiac sound signal for the period during which the subject is asleep. In this sleep data, reference numeral 16 designates a large amplitude signal due to movements of the subject; reference numeral 17 designates the cardiac sound signal; and reference numeral 18 designates a mute state. Cardiac sound signal 17 includes information comprised of a plurality of different amplitudes.

Each of the foregoing signals indicates the following state, respectively. Large amplitude signal 16 represents movements and roll-over or contraction of muscles of the subject. Cardiac sound signal 17 having a constant amplitude, which lasts relatively long, represents a deep sleep at sleep stage 3 to 4. A period in which large amplitude signal 16 appears relatively many times in cardiac sound signal 17 represents a shallow sleep at sleep stage 1 to 2 or REM sleep. In this connection, variations in the amplitude of cardiac sound signal 17 represent changes in posture or movements of the body. Mute signal 18 indicates that the body of the subject has largely moved out of the detectable area of heartbeat/respiration sensor 4, in which case it is thought that the subject has fallen down from the bed, or has left the bed and is moving about. Accordingly, the state of sleep can be simply determined by reading these signals from the captured sleep data.

According to heartbeat/respiration sensor 4 and the body monitor using the same described above, it is possible to avoid a variety of obstacles which could be otherwise caused by electrodes of a electrocardiograph being attached to a newborn infant, and additionally save the effort of attaching the electrodes of the electrocardiograph, so that the newborn infant can be immediately monitored for required heartbeat, respiration and the like in a completely non-invasive manner without causing discomfort to the newborn infant. Actually, electrocardiography is not required except for seriously ill newborn infants, but it is often the case that the monitor of this embodiment suffices for monitoring. Further, since highly sensitive piezoelectric transducer 2 is used in heartbeat/respiration sensor 4, the embodiment can provide effects similar to those provided by auscultation of the newborn infant at all times, which is difficult to perform within an incubator. Actually, cardiac noise can also be confirmed on monitor 14.

Also, since heartbeat/respiration sensor 4 of this embodiment has a smooth surface and a low profile, it can be readily inserted below the body of a newborn infant even after the newborn infant has been placed in an incubator. Piezoelectric transducer 2 per se is highly sensitive and is capable of detecting vital signals, and thin vibration transmitting plate 1 further serves to extend a range in which vital signals can be detected by piezoelectric transducer 2. Thus, even if heartbeat/respiration sensor 4 is wrapped with towel 6 or the like, or even if a subject remains with his clothes on, or even if heartbeat/respiration sensor 4 is installed on a soft comforter, vital signals can be highly sensitively detected. Also, since heartbeat/respiration sensor 4 of the embodiment is in a simple configuration, basically made up of piezoelectric transducer 2 and vibration transmitting plate 1 to which piezoelectric sensor 2 is attached, the manufacturing cost thereof can be kept low. Accordingly, a wide variety of applications can be contemplated for heartbeat/respiration sensor 4 so that the marketability of heartbeat/respiration sensor 4 of the embodiment can be widely acquired.

Heartbeat/respiration sensor 4 of the embodiment and the body monitor using the same can be used not only for newborn infants but also for adults, elderly persons, and cared persons without modifying the configuration. Accordingly, the body monitor using heartbeat/respiration sensor 4 can be provided at a low cost and in a convenient manner, unlike complicated systems so far proposed, as a health care tool (for example, a device for monitoring a sleeping state and a sleeping pattern) available for home use, which is now increasingly more required. Further, the body monitor of the embodiment can be applied to health care for cared persons, and for confirmation that elderly persons are moving about, and the like. As further applications, heartbeat/respiration sensor 4 can be attached to a driver's seat in a variety of traveling means (car, train, aircraft and the like) to monitor the driver for a health condition, or heartbeat/respiration sensor 4 can be attached to the back of a patient chair in the examination room of a hospital to examine the patient without requiring the patient to take off closes.

## Claims

1. A heartbeat/respiration sensor comprising:
a flexible vibration transmitting plate formed with a plurality of vent holes therethrough; and
a piezoelectric transducer mounted on said vibration transmitting plate.

2. The heartbeat/respiration sensor according to claim 1, wherein said vibration transmitting plate has rounded corners.

3. The heartbeat/respiration sensor according to claim 1 or 2, wherein an alignment line is drawn on said vibration transmitting plate for indicating the position at which said piezoelectric transducer is mounted.

4. The heartbeat/respiration sensor according to any one of claims 1 to 3, comprising a cover made of a terry cloth and encasing said sensor, wherein an alignment line is drawn on said cover for indicating the position of said piezoelectric transducer when said cover encases said heartbeat/respiration sensor.

5. A body monitor comprising:
the heartbeat/respiration sensor according to any one of claims 1 to 4;
a filter circuit for separating a cardiac sound signal and a respiration signal from an output signal of said heartbeat/respiration sensor; and
a monitor for displaying the cardiac sound signal and the respiration signal.
